# EUROPEAN PATENT APPLICATION

(11) **EP 3 085 342 A1**
(43) Date of publication of application: **26.10.2016**
(21) Application number: 14871622.8
(22) Date of filing: 15.12.2014
(51) Int. Cl.: A61F 5/01, A47C 7/14, A47C 9/00

(54) **CHAIR**

(30) Priority: 17.12.2013 JP 2013260207
(71) Applicant: Microdesign Limited, Tokyo 144-0052 (JP)
(72) Inventor: NISHIMIYA, Yuki, Tokyo 144-0052 (JP)
(74) Representative: Lecomte & Partners
(86) International application number: PCT/JP2014/006231
(87) International publication number: WO 2015/093033

(57) **Abstract**

To provide a chair such that, when a person sits thereon, a force in the direction that widens the pelvis to the left and right does not act on the pelvis, and a function of sandwiching the pelvis from both the left and right sides is exerted naturally once a person sits on the chair, left and right pad members (20) are attached to an upper portion of a leg part (16), and pad surfaces (26) of the left and right pad members (20) that oppose each other face each other and are inclined so as to be closer to each other in a downward direction such that the pad surfaces come into contact with portions of a person in a sitting posture that correspond to left and right sides of the pelvis of the person, and wherein the left and right pad members (20) support an upper limb weight of a person in a sitting posture without supporting the pair of ischia from below.

## Description

### TECHNICAL FIELD

The present invention relates to a chair, and particularly relates to a chair that is effective in correcting the pelvis as well as preventing and reducing lower back pain.

### BACKGROUND ART

As a chair effective in correcting the pelvis as well as preventing and reducing lower back pain, a chair is known in which cushions that can be inflated with fluid are provided on the left and right sides of a seat plate member, respectively, such that the cushions can be brought into close contact with a pelvis portion by introducing the fluid into the cushions to thereby inflate the cushions (see Patent Document 1, for example).

Also, as a chair that causes less fatigue to a person even if the person sits on the chair for an extended period of time, a chair is known in which the seat plate for supporting the buttocks is divided into left and right seat plates in such that they can be moved in the left and right direction, whereby when a person sits on the chair, the seating load causes the left and right seat plates to move in directions away from each other to disperse the pressing force acting on the pelvis (see Patent Documents 2 and 3, for example).

### PRIOR ART DOCUMENT(S)

### PATENT DOCUMENT(S)

Patent Document 1: JP2002-360376A
Patent Document 2: JP4546957B
Patent Document 3: WO03/034870A1

### BRIEF SUMMARY OF THE INVENTION

### TASK TO BE ACCOMPLISHED BY THE INVENTION

A conventionally known chair includes a seat plate for receiving the buttocks, and supports the upper limb weight of a person in a sitting posture by supporting the pair of ischia from below with the seat plate. Thus, in a state where a person is sitting on the chair, the upper limb weight acts on the pelvis as a force in the direction that widens the pelvis to the left and right. Therefore, when a person sits on the chair, particularly when a person continues to sit on the chair for an extended period of time, the pelvis of the person is widened; namely, sitting on the chair can be a cause of lateral asymmetry (distortion) of the pelvis and/or lower back pain.

The chair in which the cushions can be brought into close contact with the pelvis portion by inflating the cushions may make the upper limb weight act less on the pelvis as a force in the direction that widens the pelvis to the left and right in a state where a person is sitting on the chair. However, the chair is no different from the conventional one in that the seat plate supports the pair of ischia from below, and therefore, it cannot prevent the upper limb weight from acting on the pelvis as a force in the direction that widens the pelvis to the left and right when a person sits on the chair.

The chair in which the left and right seat plates move in directions away from each other when applied a seating load may disperse the pressing force acting on the pelvis when a person sits on the chair. However, because the chair is configured to support the pair of ischia from below with the seat plates, the chair also cannot prevent the upper limb weight from acting on the pelvis as a force in the direction that widens the pelvis to the left and right when a person sits on the chair.

It is known that tightening the pelvis to prevent widening of the pelvis is effective in preventing or reducing lower back pain. One known device to tighten the pelvis and prevent widening of the pelvis is a pelvis belt.

A task to be accomplished by the present invention is to provide a chair such that, when a person sits thereon, a force in the direction that widens the pelvis to the left and right does not act on the pelvis, and a function of sandwiching the pelvis from both the left and right sides to tighten the pelvis is exerted once a person sits on the chair.

### MEANS TO ACCOMPLISH THE TASK

A chair (10, 30, 80, 110) according to the present invention is configured to include: a leg part (16, 36, 82, 112); and a pad member (20, 54, 84, 116) mounted to an upper portion of the leg part (16, 36, 82, 112), wherein the pad member (20, 54, 84, 116) includes left and right pad surfaces (26, 60, 90, 122) which face each other and are inclined so as to be closer to each other in a direction from up to down such that the pad surfaces come into contact with portions of a person in a sitting posture that correspond to left and right sides of a pelvis of the person, and wherein the pad member (20, 54, 84, 116) supports the upper limb weight of the person sitting on the chair without supporting a pair of ischia of the person from below.

According to this configuration, because the chair is configured not to support the pair of ischia from below, when a person sits on the chair (10, 30, 80, 110), a force in the direction that widens the pelvis (B) to the left and right is prevented from acting on the pelvis (B), and a person only has to sit on the chair for a function of sandwiching the pelvis (B) from both the left and right sides to tighten the pelvis (B) to be exerted.

In the chair (10, 30, 80, 110) according to the present invention, preferably, a lower side of the left and right pad members (20, 54, 84, 116) is opened.

According to this configuration, when a person sits on the chair (10, 30, 80, 110), the chair does not support the pair of ischia from below, and a force in the direction that widens the pelvis (B) to the left and right does not act on the pelvis (B) in a state where a person is sitting on the chair.

In the chair (30) according to the present invention, preferably, the left and right pad surfaces (26, 60, 90, 122) are also inclined so as to be more distant from each other in a direction from rear to front.

According to this configuration, the lateral distance between the left and right pad surfaces (26, 60, 90, 122) increases in the frontward direction, and this can avoid restraining of the hip joint portion when a person sits on the chair.

In the chair (30) according to the present invention, preferably, the pad member (54) is constituted of left and right individual pad members (54) and is mounted to the leg part (30) via a position adjustment mechanism (62, 68, 70) capable of changing positions of the pad members in a direction to vary a distance between the left and right pad surfaces (60).

According to this configuration, the position adjustment mechanism (62, 68, 70) enables adjustment of the separation distance between the left and right pad surfaces (60), and this makes the chair adaptable to a wide range of people with varying builds.

In the chair (30) according to the present invention, preferably, the pad member (54) is mounted to the leg part (30) via a coupling mechanism (64) capable of fixedly securing the pad member with an arbitrary inclined attitude.

According to this configuration, the coupling mechanism (64) enables the pad surfaces (60) to take an arbitrary inclined attitude, and this makes the chair adaptable to a wide range of people with varying builds.

Preferably, the chair (30) according to the present invention further includes left and right backrest halves (92) which extend upward from rear end portions of the left and right pad members (84), respectively, and are inclined so as to be more distant from each other in a direction from laterally inside to outside, the backrest halves in cooperation serving as one backrest.

According to this configuration, the back of a person leaning against the backrest halves (92) can be held stably without a feel of pressure on the spine, and this makes the chair more comfortable to sit on.

### EFFECT OF THE INVENTION

According to the chair of the present invention, since the chair is configured not to support the pair of ischia from below, when a person sits on the chair, a force in the direction that widens the pelvis to the left and right is prevented from acting on the pelvis, and a person only has to sit on the chair for a function of sandwiching the pelvis from both the left and right sides to tighten the pelvis to be exerted.

### BRIEF DESCRIPTION OF THE DRAWINGS

FIG. 1 is a front view showing the first embodiment of a chair according to the present invention;
FIG. 2 is a plan view of the chair of the first embodiment;
FIG. 3 is a perspective view showing the second embodiment of the chair according to the present invention;
FIG. 4 is a front view in which main parts of the chair of the second embodiment are shown in partial cross-section;
FIG. 5 is a front view showing the third embodiment of the chair according to the present invention;
FIG. 6 is a perspective view of the chair of the third embodiment;
FIG. 7 is a front view showing the fourth embodiment of the chair according to the present invention; and
FIG. 8 is a perspective view of the chair of the fourth embodiment.

### EMBODIMENTS FOR CARRYING OUT THE INVENTION

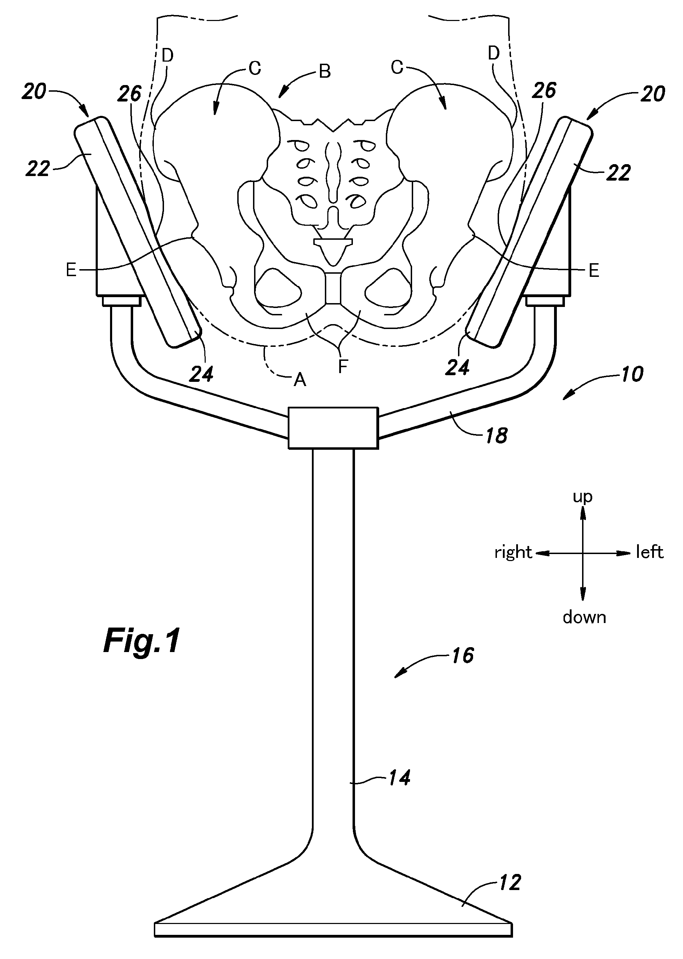

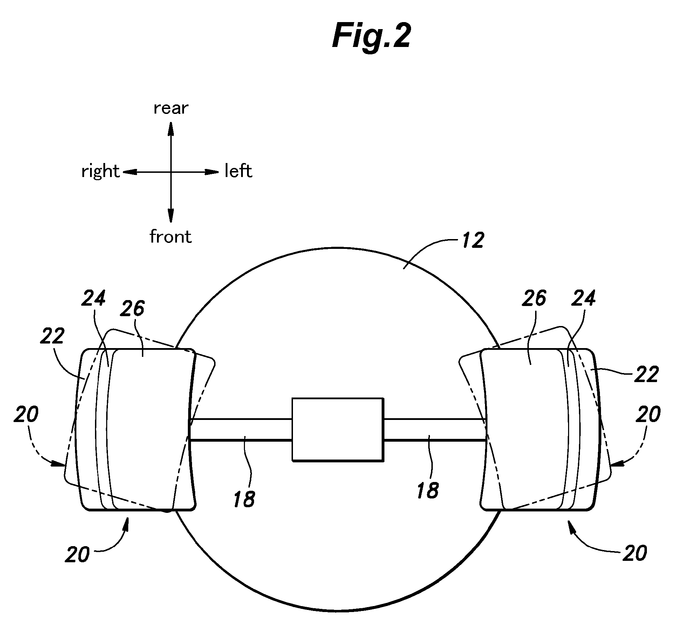

In the following, the first embodiment of a chair according to the present invention will be described with reference to FIGS. 1 and 2.

The chair 10 of the first embodiment is designed as a stool, and includes a metallic leg part (leg body) 16 formed of a disk-shaped base 12 to be placed on a floor and a pole 14 extending vertically from a central part of the base 12. Fixedly attached to an upper portion of the leg part 16 is an upper support member 18 made of a metallic pipe. The upper support member 18 extends on left and right sides of the pole 14 so as to have a substantially U-shape as viewed from the front.

Left and right pad members 20 are attached to the left and right ends of the upper support member 18, respectively. The left and right pad members 20 each include a rectangular base plate 22 made of metal or synthetic resin and a cushion member 24 attached to one face of the base plate 22 to cover the entirety of the face, and are each curved in an inwardly concave arc shape when seen in plan view, such that the pad members 20 extend along the respective side faces of the buttocks of a person. The left and right cushion members 24 are each made of resilient material such as foamed urethane resin in a shape of a sheet having a uniform thickness.

The base plate 22 of each of the left and right pad members 20 is fixedly secured to a corresponding end of the upper support member 18 such that the cushion members 24 of the pad members 20 face each other and are arranged in a truncated V-shape as viewed from the front. In this arrangement, the mutually facing surfaces of the cushion members 24 are referred to as pad surfaces 26. The left and right pad surfaces 26 face each other and are inclined so as to be closer to each other in the direction from up to down. Namely, the lateral separation distance between the left and right pad surfaces 26 decreases gradually in the direction from up to down.

The inclination angle of each pad surface 26 relative to the horizontal plane may be about 60 degrees. The lateral separation distance between the left and right pad surfaces 26 is larger than the width of the buttocks (pelvis) of a standard adult at upper ends thereof and is smaller than the width of the buttocks A (pelvis B) of a standard adult at lower ends thereof Owing to these settings, when a person sits on the chair by inserting the buttocks between the left and right pad surfaces 26 from the upper side to the lower side, the left and right pad surfaces 26 naturally come to contact, with at least a part thereof in the vertical direction, the portions of the person in the sitting posture that correspond to the left and right sides of the pair of hipbones C of the pelvis B, particularly the left and right sides of the pair of ilia (anterior superior iliac spine D to anterior inferior iliac spine E).

The lower ends and lower side of the left and right pad members 20 are opened, and there is no seat plate nor a member equivalent thereto that supports the pair of ischia F from below. Thereby, the pair of ischia F of a person sitting on the chair 10 is not supported from below.

With this chair 10, when an adult person assumes a sitting posture by inserting the buttocks between the left and right pad surfaces 26 from the upper side to the lower side, the left and right pad surfaces 26 respectively contact the portions of the person that correspond to the left and right sides of the pair of hipbones C. Thereby, the pad members 20 sandwich the portions of the person corresponding to the pair of hipbones C from left and right to thereby support the upper limb weight of the adult person in the sitting posture without supporting the pair of ischia F from below. At this time, the cushion members 24 deform resiliently to conform to the shape of the side portions of the buttocks and hold the side portions of the buttocks stably.

Thereby, when a person sits on the chair 10, the upper limb weight is prevented from acting on the pelvis B as a force in the direction that widens the pelvis B to the left and right, and once a person sits on the chair, the left and right pad surfaces 26 naturally exert a function of sandwiching the pelvis B from both the left and right sides, such that the pelvis B is tightened laterally inward by the horizontal components of force derived from the upper limb weight acting on the left and right pad surfaces 26. Owing to this function, a person only has to sit on the chair 10 and correction of distortion of the pelvis B is performed effectively, contrary to inducing lateral asymmetry to the pelvis B, and in addition, an effect of preventing or reducing lower back pain and sciatica is obtained.

Further, in the chair 10, since the lower side of the left and right pad members 20 is opened and there is no seat plate supporting the pair of ischia F from below, an affected area of a patient who has undergone a surgery for hemorrhoid or who is with anal fistula is not pressed, and therefore, a person with such a disease can assume a sitting posture easily without pain. Further, the above structure enables a pregnant woman to assume a sitting posture.

In this embodiment, a person only has to sit on the chair 10 to have the left and right pad surfaces 26 naturally sandwich the pelvis B from both the left and right sides, and only has to stand up to release the pelvis B from between the left and right pad surfaces 26. Therefore, the chair 10 does not compromise the ease of sitting down and standing up and does not deteriorate the usability as a chair. It is to be noted that "sitting on the chair" in the description of the embodiments does not mean putting the pair of ischia F on the seat plate nor a member equivalent thereto, but assuming a posture sitting on the chair.

In addition to being arranged in a truncated V-shape as viewed from the front, the left and right pad members 20 (the pad surfaces 26) may be arranged, as shown by phantom lines in FIG. 2, such that the front side opens wider than the rear, namely, in an inverted truncated V-shape as seen in plan view such that the lateral separation distance between the left and right pad surfaces 26 increases in the frontward direction. In other words, the left and right pad surfaces 26 may be arranged such that they are inclined so as to be more distant from each other in the direction from rear to front. Owing to this arrangement, the hip joint portion of the pelvis B sandwiched by the left and right pad surfaces 26 is not too restrained, and the feeling of pressure perceived by a person sitting on the chair can be alleviated.

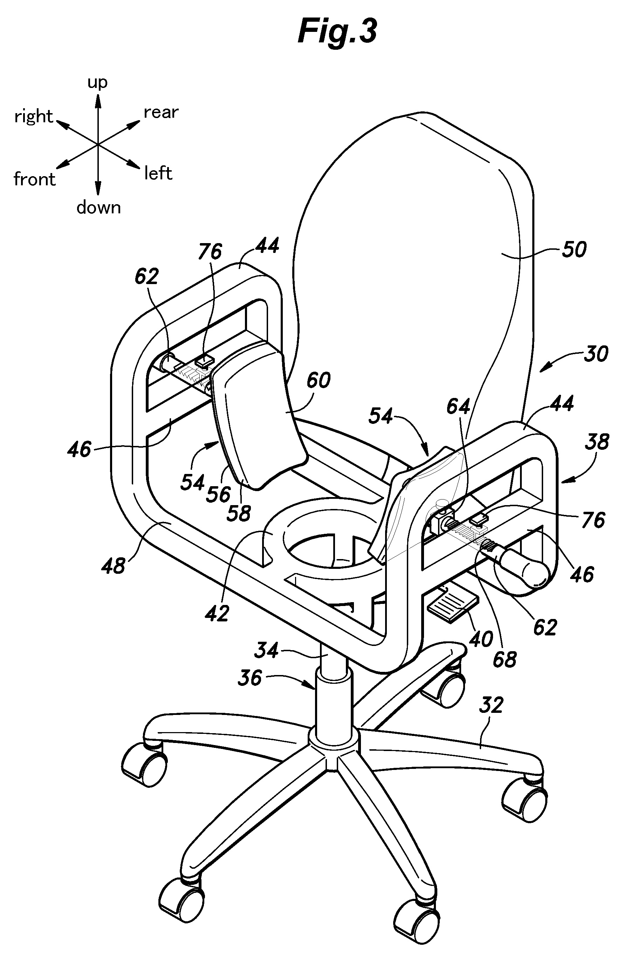

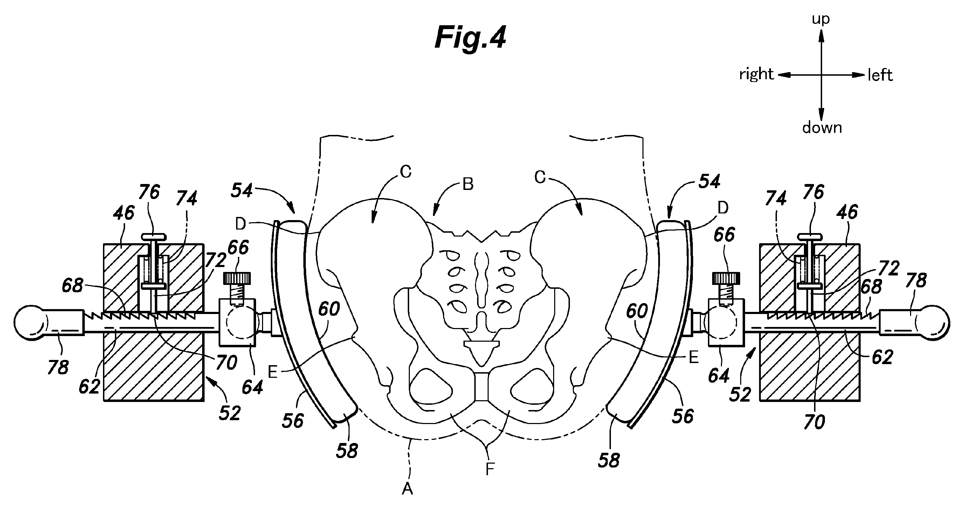

Next, description will be made of the second embodiment of the chair according to the present invention with reference to FIGS. 3 and 4.

The chair 30 of the second embodiment is designed as an office chair, and includes a leg part (leg body) 36 constituted of a base 32, which has five lets each provided with a caster and is to be placed on the floor, and a pole 34 extending vertically from a central part of the base 32. Fixedly attached to an upper portion of the leg part 36 is an upper support member 38. The pole 34 includes a gas-filled damper (not shown in the drawings), such that the height of the upper support member 38 relative to the floor can be adjusted by use of an adjustment lever 40 of the gas-filled damper.

The upper support member 38 includes a frame member 48 constituted of a round cage-like portion 42 fixedly secured to an upper end of the pole 34, left and right armrests 44 attached to the round cage-like portion 42, and left and right side beams 46 respectively extending horizontally in the front and rear direction below the corresponding armrests 44. A backrest member 50 is attached to a rear side of the frame member 48.

Left and right pad members 54 are attached to the left and right side beams 46 via pad supporting mechanisms 52, respectively. The left and right pad members 54 each include a rectangular base plates 56 made of metal or synthetic resin and a cushion member 58 attached to one face of the base plate 56 to cover the entirety of the face, and are each curved in an inwardly concave arc shape when seen both in plan view and in front view, such that the pad members 54 extend along the respective side faces of the buttocks of a person. The left and right cushion members 58 are each made of resilient material such as foamed urethane resin in a shape of a sheet having a uniform thickness, and the mutually facing surfaces of the cushion members 58 serve as pad surfaces 60.

Each pad supporting mechanism 52 includes one of left and right horizontal rod 62 attached to the respective side beams 46 so as to be moveable in the left and right direction and a spherical joint 64 attached to an inner end of the corresponding one of the left and right horizontal rods 62, and the base plate 56 of the pad member 54 on the corresponding side is attached to the spherical joint 64.

Each spherical joint 64 serves as a coupling mechanism with a lock that can be fixedly secured with an arbitrary variable inclined attitude, and consists of a spherical joint with a lock configured to fixedly secure the pad member 54 to the end of the horizontal rod 62 with an arbitrary inclined attitude by means of a lock screw 66. Thereby, the pad member 54 is fixedly secured to the end of horizontal rod 62 such that the inclination angle relative to the vertical direction and the inclination angle relative to the front and rear direction, namely, the inclination angle in any direction can be varied.

Each horizontal rod 62 is provided with a series of ratchet teeth 68 along its axial direction (left and right direction). The side beam 46 is provided with a ratchet pawl 70 that releasably engages one of the ratchet teeth 68, a stem 72 having one end to which the ratchet pawl 70 is attached, a compression coil spring 74 that urges the ratchet pawl 70 into engagement with the ratchet teeth 68, and a ratchet release knob 76 attached to the other end of the stem 72 such that the knob 76 is manipulated when releasing the ratchet pawl 70 from the engagement with the ratchet teeth 68. Attached to an outer end of the horizontal rod 62 is a rod manipulation knob 78 for moving the horizontal rod 62 manually in the axial direction.

The ratchet mechanism constituted of the ratchet teeth 68 and the ratchet pawl 70 serves as a one-way locking mechanism that prevents the movement of the horizontal rod 62 outward in the axial direction (movement in the direction that enlarges the lateral separation distance between the left and right pad surfaces 60) and permits the movement of the horizontal rod 62 inward in the axial direction. Thus, a user can place the pad members 54 at an arbitrary lateral position by gripping the rod manipulation knob 78 and manually moving the horizontal rod 62 inward in the axial direction, or by releasing the ratchet engagement by use of the ratchet release knob 76 and manually moving the horizontal rod 62 outward in the axial direction. In this way, a position adjustment mechanism is constituted, such that the lateral separation distance between the left and right pad surfaces 60 can be variably set.

In this embodiment also, the lower side of the left and right pad members 54 is opened, and there is no seat plate nor a member equivalent thereto that supports the pair of ischia F from below.

In this embodiment, in accordance with the build of the user, the lateral separation distance between the left and right pad surfaces 60 and the inclination angles of the pad members 54 relative to the vertical direction and relative to the front and rear direction are adjusted such that the lateral separation distance between the left and right pad surfaces 60 is larger than the width of the buttocks (the pelvis B) at upper ends thereof and is smaller than the width of the buttocks A (the pelvis B) at lower ends thereof, whereby when a person sits on the chair by inserting the buttocks between the left and right pad surfaces 60 from the upper side to the lower side, the left and right pad surfaces 60 come to contact the portions of the person in the sitting posture that correspond to the left and right sides of the pair of hipbones C of the pelvis B, particularly the left and right sides of the pair of ilia (anterior superior iliac spine D to anterior inferior iliac spine E).

By the adjustment of inclination of the left and right pad surfaces 60 relative to the vertical direction, the pad surfaces 60 are inclined so as to be closer to each other in the direction from up to down, and by the adjustment of inclination of the same relative to the front and rear direction, the pad surfaces 60 are inclined so as to be more distant from each other in the direction from rear to front. Thereby, the left and right pad surfaces 60 are arranged in a truncated V-shape as viewed from the front and in an inverted truncated V-shape as seen in plan view such that the front side opens wider than the rear.

With this chair 30 also, when an adult person assumes a sitting posture by inserting the buttocks between the left and right pad surfaces 60 from the upper side to the lower side, the left and right pad surfaces 60 respectively contact the portions of the person that correspond to the left and right sides of the pair of hipbones C. Thereby, the pad members 54 sandwich the portions of the person corresponding to the left and right sides of the pair of hipbones C from left and right to thereby support the upper limb weight of the adult person in the sitting posture without supporting the pair of ischia F from below. At this time, the cushion members 58 deform resiliently to conform to the shape of the side portions of the buttocks and hold the side portions of the buttocks stably.

Thereby, when a person sits on the chair 30, the upper limb weight is prevented from acting on the pelvis B as a force in the direction that widens the pelvis B to the left and right, and once a person sits on the chair, the left and right pad surfaces 60 naturally exert a function of sandwiching the pelvis B from both the left and right sides, such that the pelvis B is tightened laterally inward by the horizontal components of force derived from the upper limb weight acting on the left and right pad surfaces 60. Owing to this function, a person only has to sit on the chair 30 and correction of distortion of the pelvis B is performed effectively, contrary to inducing lateral asymmetry to the pelvis B, and in addition, an effect of preventing or reducing lower back pain and sciatica is obtained.

Further, in the chair 30 also, since the lower side of the left and right pad members 54 is opened and there is no seat plate supporting the pair of ischia F from below, an affected area of a patient who has undergone a surgery for hemorrhoid or who is with anal fistula is not pressed, and therefore, a person with such a disease can assume a sitting posture easily without pain. Further, the above structure enables a pregnant woman to assume a sitting posture.

With the chair 30 also, once a person sits thereon, the left and right pad surfaces 60 naturally sandwich the pelvis B from both the left and right sides, and the person only has to stand up to release the pelvis B from between the left and right pad surfaces 60. Therefore, this chair does not compromise the ease of sitting down and standing up and does not deteriorate the usability as a chair.

In this chair 30, it is possible to increase and decrease the separation distance between the left and right pad surfaces 60. Further, since the pad members 54 can take an arbitrary attitude, the chair can be adapted to a wide range of people with varying builds.

Further, because the left and right pad surfaces 60 are arranged in an inverted truncated V-shape as seen in plan view such that the front side opens wider than the rear, namely, are inclined so as to be more distant from each other in the direction from rear to front, the hip joint portion of the pelvis B sandwiched by the left and right pad surfaces 60 is not too restrained, and the feeling of pressure perceived by a person sitting on the chair can be alleviated.

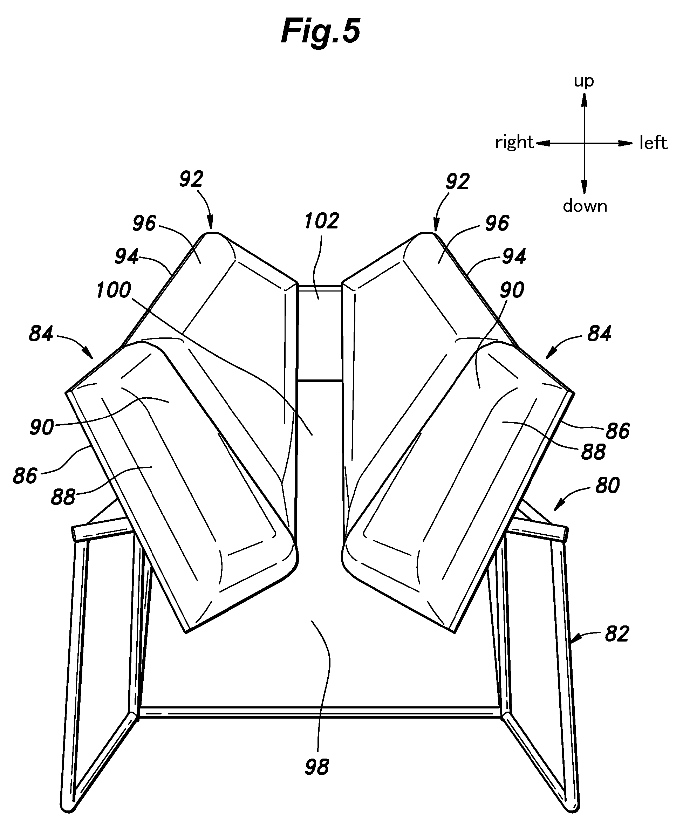

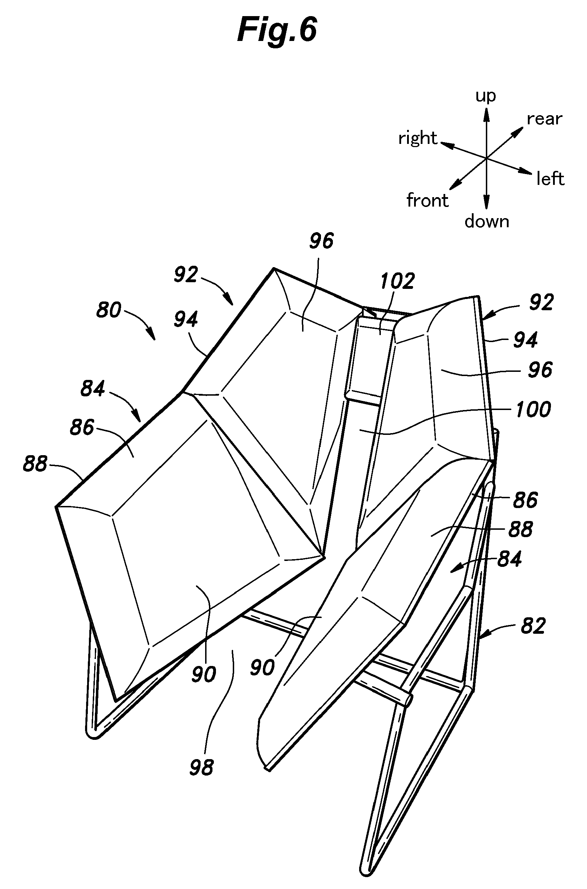

Next, description will be made of the third embodiment of the chair according to the present invention with reference to FIGS. 5 and 6.

The chair 80 of the third embodiment is designed as a guest chair, and includes a frame-shaped leg body (leg part) 82 to be placed on the floor and left and right pad members 84 fixedly secured on top of the leg body 82. The left and right pad members 84 each include a rectangular base plate 86 made of metal or synthetic resin and elongated in the front and rear direction and a cushion member 88 attached to one face of the base plate 86 to cover the entirety of the face. The left and right cushion members 88 are each made of resilient material such as foamed urethane resin and have a uniform thickness except for a peripheral part.

The left and right pad members 84 are fixedly secured to the leg body 82 such that the cushion members 88 face each other and are arranged in a truncated V-shape as viewed from the front and in an inverted truncated V-shape as seen in plan view such that the front side opens wider than the rear. In this arrangement, the mutually facing surfaces of the cushion members 88 are referred to as pad surfaces 90. The left and right pad surfaces 90 face each other and are not only inclined so as to be closer to each other in the direction from up to down but are also inclined so as to be more distant from each other in the direction from rear to front. Namely, the lateral separation distance between the left and right pad surfaces 90 decreases gradually in the direction from up to down and decreases gradually in the direction from front to rear. The lower side of the left and right pad members 84 is opened, and there is no seat plate nor a member equivalent thereto that supports the pair of ischia F from below.

In this embodiment also, the lateral separation distance between the left and right pad surfaces 90 is larger than the width of the buttocks (pelvis) of a standard adult at upper ends thereof and is smaller than the width of the buttocks A (pelvis B) of a standard adult at lower ends thereof Owing to these settings, when a person sits on the chair by inserting the buttocks between the left and right pad surfaces 90 from the upper side to the lower side, the left and right pad surfaces 90 naturally come to contact, with at least a part thereof in the vertical direction, the portions of the person in the sitting posture that correspond to the left and right sides of the pair of hipbones C of the pelvis B, particularly the left and right sides of the pair of ilia (anterior superior iliac spine D to anterior inferior iliac spine E).

Also mounted to the leg body 82 are left and right individual backrest halves 92. The backrest halves 92 are provided in a left and right pair and cooperate with each other to serve as one backrest. Each of the backrest halves 92 includes a rectangular flat base plate 94 made of metal or synthetic resin and a cushion member 96 attached to one face of the base plate 94 to cover the entirety of the face. The cushion members 96 are each made of resilient material such as foamed urethane resin and have a uniform thickness except for a peripheral part. The lower end portion of each backrest half 92 extends in an upwardly bent manner from the rear end portion of a corresponding one of the left and right pad members 84 such that the backrest half 92 extends obliquely upward in the rearward direction.

The left and right backrest halves 92 are inclined so as to be more distant from each other in the direction from laterally inside to outside and are thereby arranged in an inverted truncated V-shape as seen in plan view such that the front side opens wider than the rear. The left and right backrest halves 92 define therebetween a slit-shaped opening 100 that is continuous to an opening 98 defined between the lower ends of the left and right pad members 84 (an opening in an inverted truncated V-shape with a wider front side). Upper end portions of the left and right backrest halves 92 are joined to each other by a strip-shaped coupling member 102. It is to be noted that the base plates 94 of the backrest halves 92 and the corresponding base plates 86 of the pad members 84 may be integral with each other.

In this chair 80 also, when an adult person assumes a sitting posture by inserting the buttocks between the left and right pad surfaces 90 from the upper side to the lower side, the left and right pad surfaces 90 respectively contact the portions of the person that correspond to the left and right sides of the pair of hipbones C (refer to FIG. 1). Thereby, the pad members 54 sandwich the portions of the person corresponding to the left and right sides of the pair of hipbones C from left and fight to thereby support the upper limb weight of the adult person in the sitting posture without supporting the pair of ischia F (refer to FIG. 1) from below. At this time, the cushion members 88 deform resiliently to conform to the shape of the side portions of the buttocks and hold the side portions of the buttocks stably.

Thereby, when a person sits on the chair 80, the upper limb weight is prevented from acting on the pelvis B (refer to FIG. 1) as a force in the direction that widens the pelvis B to the left and right, and once a person sits on the chair, the left and right pad surfaces 90 naturally exerts a function of sandwiching the pelvis B from both the left and right sides, such that the pelvis B is tightened laterally inward by the horizontal components of force derived from the upper limb weight acting on the left and right pad surfaces 90. Owing to this function, a person only has to sit on the chair 80 and correction of distortion of the pelvis B is performed effectively, contrary to inducing lateral asymmetry to the pelvis B, and in addition, an effect of preventing or reducing lower back pain and sciatica is obtained.

Further, in the chair 80 also, since the lower side of the left and right pad members 84 is opened by the opening 98 and there is no seat plate supporting the pair of ischia from below, an affected area of a patient who has undergone a surgery for hemorrhoid or who is with anal fistula is not pressed, and therefore, a person with such a disease can assume a sitting posture easily without pain. Further, the above structure enables a pregnant woman to assume a sitting posture.

With the chair 80 also, when a person sits thereon, the left and right pad surfaces 90 naturally sandwich the pelvis B from both the left and right sides, and the person only has to stand up to release the pelvis B from between the left and right pad surfaces 90. Therefore, this chair does not compromise the ease of sitting down and standing up and does not deteriorate the usability as a chair.

Further, in this embodiment also, the left and right pad surfaces 90 are arranged in an inverted truncated V-shape such that the front side opens wider than the rear, namely, are inclined so as to be more distant from each other in the direction from rear to front, and therefore, the hip joint portion of the pelvis B sandwiched by the left and right pad surfaces 90 is not too restrained, and the feeling of pressure perceived by a person sitting on the chair can be alleviated.

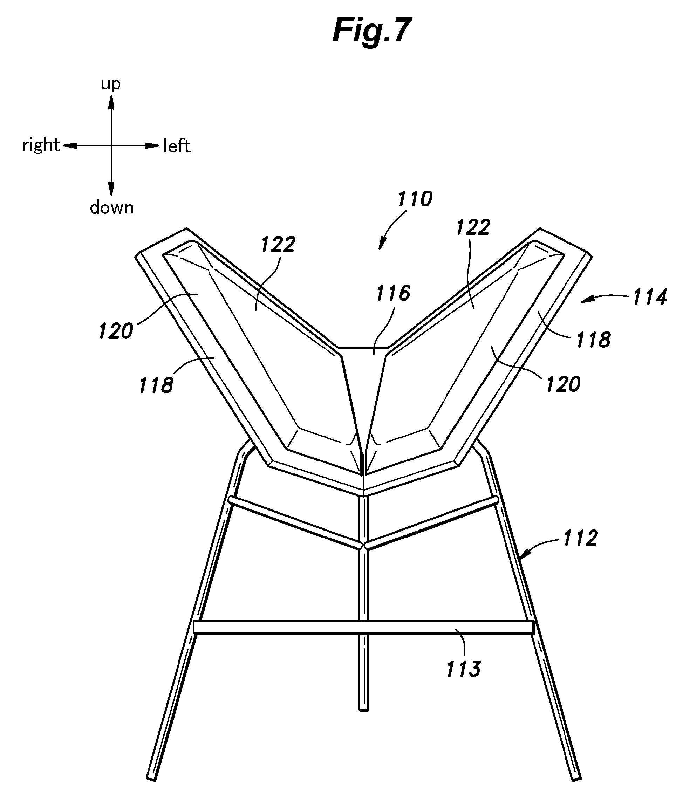

As in the embodiment shown in FIG. 6 and FIG. 7, in the case where the left and right pad surfaces 60 are inclined so as to be more distant from each other in the direction from rear to front and the pad surfaces 90 have a large front-rear dimension, by changing the position of the person sitting on the chair 80 in the front and rear direction, namely, by sitting shallow or sitting back on the chair, it is possible to adjust the degree of tightening caused by sandwiching of the pelvis B by the left and right pad surfaces 60 from both the left and right sides. If one desires to increase the degree of tightening caused by sandwiching of the pelvis B by the left and right pad surfaces 60 from both the left and right sides, the one may sit back on the chair and if one desires to decrease the degree of tightening, the one may sit shallow on the chair.

Because the left and right backrest halves 92 are inclined so as to be more distant from each other in the direction from laterally inside to outside and are thereby arranged in an inverted truncated V-shape as seen in plan view such that the front side opens wider than the rear, the back of a person leaning on the backrest halves 92 can be held stably without a feel of pressure on the spine. This makes the chair more comfortable to sit on.

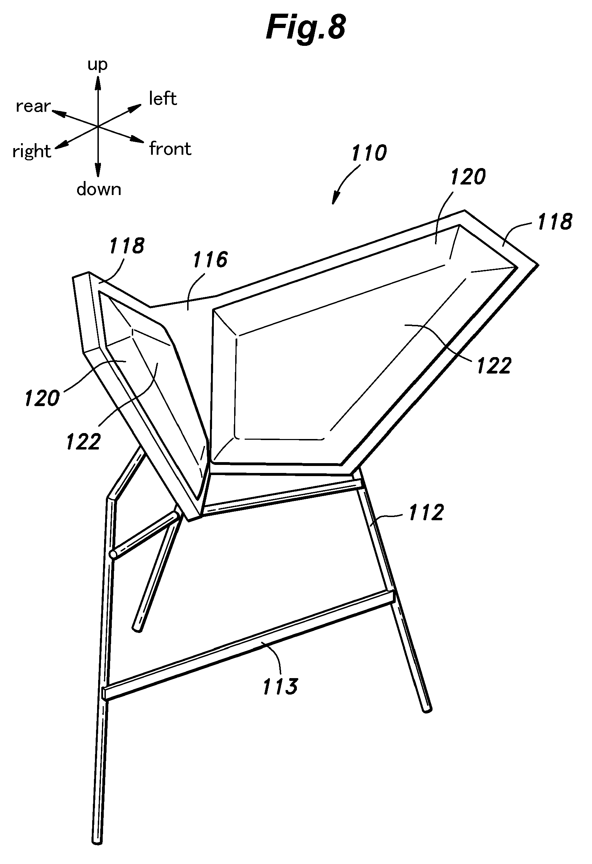

Next, description will be made of the fourth embodiment of the chair according to the present invention with reference to FIGS. 7 and 8.

The chair 110 of the fourth embodiment is designed as a stool, and includes a tripod-like leg body (leg part) 112 to be placed on the floor. A footrest bar 113 is attached to a front portion of the leg bodyl12.

Fixedly secured to an upper portion of the leg body (leg part) 112 is a base plate 116 of a pad member114. The base plate 116 is made of metal or synthetic resin, and is bent to be in a truncated V-shape as viewed from the front and in an inverted truncated V-shape as seen in plan view such that the front side opens wider than the rear. The base plate 116 has a pair of symmetrically arranged pentagonal pad-mounting planar parts 118, and a pentagonal cushion member 120 is attached to each of the left and right pad-mounting planar parts 118.

The mutually facing surfaces of the cushion members 120 are referred to as pad surfaces 122, and the left and right pad surfaces 122 are each inclined in two directions so as to be closer to each other in the direction from up to down and more distant from each other in the direction from rear to front. Namely, the lateral separation distance between the left and right pad surfaces 122 decreases gradually in the direction from up to down and increases gradually in the direction from rear to front. In other words, the left and right pad surfaces 122 are arranged in a truncated V-shape as viewed from the front and in an inverted truncated V-shape as seen in plan view such that the front side opens wider than the rear.

Thereby, with this chair 110 also, the functions and effects same as those of the chair 80 of the third embodiment can be obtained except for the functions and effects relating to the backrest. The chair 110 allows a person to put the legs on the footrest bar 113 so that the person can sit on the chair stably.

The present invention has been described in the foregoing in terms of preferred embodiments thereof. However, as is apparent to a person having ordinary in the art, the present invention is not limited by the foregoing embodiments, and various alterations and modifications are possible without departing from the spirit of the present invention.

For instance, the pad members 20 may be connected to each other on the rear side thereof or at the lower ends so that they are in a V-shape or in an inverted trapezoidal shape as viewed from the front, so long as they do not support the pair of ischia from below in a normal state. The coupling mechanism for fixedly securing each pad member 54 with an arbitrary variable inclined attitude is not limited to the spherical joint 64, and when the adjustment of the inclined attitude is required to be performed only in one direction, the coupling mechanism may be implemented by a shaft member that rotatably supports the pad member 54 and a lock screw for fixedly securing the pad member 54 to the shaft member at an arbitrary rotation position.

Further, not all of the structural elements shown in the foregoing embodiments are necessarily indispensable, and they may be selectively used as appropriate without departing from the spirit of the present invention.

The contents disclose in the original Japanese patent application (Japanese Patent Application No. 2013-260207 filed on December 17, 2013) on which the Paris Convention priority claim is made for the present application are incorporated herein in their entirety by reference.

### GLOSSARY

- 10: chair
- 12: base
- 14: pole
- 16: leg part
- 18: upper support member
- 20: pad member
- 22: base plate
- 24: cushion member
- 26: pad surface
- 30: chair
- 32: base
- 34: pole
- 36: leg part
- 38: upper support member
- 40: adjustment lever
- 42: round cage-like portion
- 44: armrest
- 46: side beam
- 48: frame member
- 50: backrest member
- 52: pad supporting mechanism
- 54: pad member
- 56: base plate
- 58: cushion member
- 60: pad surface
- 62: horizontal rod
- 64: spherical joint
- 68: ratchet teeth
- 70: ratchet paw1
- 72: stem
- 74: compression coil spring
- 76: ratchet release knob
- 78: rod manipulation knob
- 80: chair
- 82: leg body
- 84: pad member
- 86: base plate
- 88: cushion member
- 90: pad surface
- 92: backrest halves
- 94: base plate
- 96: cushion member
- 98: opening
- 100: opening
- 102: coupling member
- 108: cushion member
- 110: chair
- 112: leg body
- 114: pad member
- 116: base plate
- 118: pad-mounting planar part
- 120: cushion member
- 122: pad surface
- A: buttocks
- B: pelvis
- C: hipbone
- F: the pair of ischia

## Claims

1. A chair comprising:
a leg part; and
a pad member mounted to an upper portion of the leg part,
wherein the pad member includes left and right pad surfaces which face each other and are inclined so as to be closer to each other in a direction from up to down such that the pad surfaces come into contact with portions of a person in a sitting posture that correspond to left and right sides of a pelvis of the person, and
wherein the pad member supports an upper limb weight of the person sitting on the chair without supporting a pair of ischia of the person from below.

2. The chair according to claim 1, wherein a lower side of the left and right pad members is opened.

3. The chair according to claim 1 or 2, wherein the left and right pad surfaces are inclined so as to be more distant from each other in a direction from rear to front.

4. The chair according to any one of claims 1 to 3, wherein the pad member is constituted of left and right individual pad members and is mounted to the leg part via a position adjustment mechanism capable of changing positions of the pad members in a direction to vary a separation distance between the left and right pad surfaces.

5. The chair according to any one of claims 1 to 4, wherein the pad member is mounted to the leg part via a coupling mechanism capable of fixedly securing the pad member with an arbitrary inclined attitude.

6. The chair according to any one of claims 1 to 5, comprising left and right backrest halves which extend upward from rear end portions of the left and right pad members, respectively, and are inclined so as to be more distant from each other in a direction from laterally inside to outside, the backrest halves in cooperation serving as one backrest.
